Europäisches Patentamt

European Patent Office

Office européen des brevets

(19)

(11) Publication number: **0 245 130**
**A1**

(12)

# EUROPEAN PATENT APPLICATION

(21) Application number: **87400746.1**

(22) Date of filing: **03.04.87**

(51) Int. Cl.⁴: **C 12 N 15/00, C 12 P 19/34**

(30) Priority: **04.04.86 EP 86400735**

(43) Date of publication of application: **11.11.87**
**Bulletin 87/46**

(84) Designated Contracting States: **AT BE CH DE ES FR GB GR IT LI LU NL SE**

(71) Applicant: **N.V. Innogenetics, Industriepark Zwijnaarde 7 Box 4, B-9710 Gent (BE)**

(72) Inventor: **Volckaert, Guido, Geestmolen Straat 24, B-3213 Holsbeek (BE)**

(74) Representative: **Gutmann, Ernest et al, S.C. Ernest Gutmann - Yves Plasseraud 67, boulevard Haussmann, F-75008 Paris (FR)**

(54) **Vectors comprising appropriate means for an enzymatical synthesis of nucleotide sequences, particularly dna fragments.**

(57) The invention relates to vectors which aim at providing suitable means for an enzymatical synthesis of DNA fragments or nucleotide sequences to be achieved.

These vectors contain in particular two tele-acting restriction zones and a determined restriction site, said two tele-acting zones comprising two cleavage sites distant from each other of one or more nucleotides.

The invention relates also to a process for elongating a nucleotide sequence of one or more determined nucleotides.

0245130

VECTORS COMPRISING APPROPRIATE MEANS FOR AN ENZYMATICAL
SYNTHESIS OF NUCLEOTIDE SEQUENCES, PARTICULARLY DNA
FRAGMENTS.

The invention relates to new vectors which comprise appropriate means enabling an enzymatical synthesis of nucleotide sequences, particularly DNA fragments, to be achieved.

It relates furthermore to a new process using these vectors for the synthesis of nucleotide sequences or fragments.

Genetic engineering generally involves directed manipulations of genetic information. These experiments can result in a complete or partial change of the primary DNA sequence. This change may be also the final goal of said experiments. Such manipulation may be carried out at the base level, e.g. site-specific mutagenesis, or at the gene level by shuffling of polynucleotide fragments. As a result, new DNA molecules can be synthesized.

Isolation and insertion of a specific number of nucleotides in a determined position, resulting in the synthesis of a new DNA fragment, can be achieved by different routes.

By using determined restriction fragments, it is possible to clone a new DNA fragment into a plasmid or a viral vector.

But if the manipulation of such DNA restriction fragments can be regarded as an act of "DNA synthesis", its final goal is not the construction of a fully predetermined primary nucleotide sequence, but rather the transfer of an already available nucleotide sequence into another sequence. The main drawback of this restriction fragment manipulation is that the base sequence at the junctions between the DNA fragment to be cloned or to be deleted and the vector will be arbitrary as it

2

results from the recognition specificities of the restriction enzymes used. The ends of the DNA fragments will usually consist of half of the enzyme's recognition sequence. Cloning of a single base-pair or fragments of two or three base-pairs long is not possible with the available cloning techniques.

Usually, however, the term "DNA synthesis" is restricted to the process of assembling nucleotides in a planned fashion.

This process of assembling separated or single nucleotides in a determined position, which results in the synthesis of a new DNA fragment, is achieved _in vitro_ generally by a chemical route.

This chemical synthesis of a DNA fragment requires the use of appropriately modified building blocks and · of a series of reactions not only to condensate the nucleotides but also to eliminate unreacted intermediates, or to remove protecting groups and finally to de-block the end-product.

This methodology, resulting in chemically synthesized oligodeoxynucleotides has been substantially improved and the process automated.

Thus, synthesis of polynucleotides of 100 nucleotides long is now feasible.

The product of chemical synthesis is a single-stranded DNA fragment. After the final de-blocking reaction, it is impossible to make any further addition by chemical means. Consequently, a complementary strand has to be separately synthesized, for producing a double-stranded DNA fragment. This complementary strand can also be prepared chemically, or, alternatively, by an enzymatic copying. For this purpose, a shorter primer, complementary to the 3' end of the polynucleotide must be chemically synthesized.

0245130

3

This chemical synthesis requires furthermore an important equipment, not always available in laboratories, and results often in an relatively high loss of product during the chemical reactions, and in a limited quantity of end-product.

The present invention provides an original solution to the above-said drawbacks. In particular, experiments followed by the applicants have shown that it was possible to synthesize DNA fragments or nucleotide sequences, particularly short ones by methods far away from the standard chemical methods recalled hereabove.

One object of the invention is to provide vectors which provide suitable means applicable to a non-chemical synthesis of DNA fragments or nucleotide sequences or to the non-chemical elongation of a DNA fragment with an oligonucleotide of any nucleotide sequence that may be sought.

A further object of the invention is to provide a non-chemical process - or "an enzymatic method" inasmuch as said process entails the use of enzymes - for the direct synthesis of single or double stranded DNA fragments or nucleotide sequences with no limitation of quantity thereof, which process can be easily carried out in all laboratories of modern biology, and with no important loss of product.

The vectors according to the invention, particularly applicable to enzymatical synthesis of a determined nucleotide sequence or fragment, are characterized by a region which comprises :

- a first tele-acting restriction zone comprising a first determined nucleotide sequence and a first blunt-ended cleavage site at a first determined distance of said first nucleotide sequence,

4

- a second tele-acting restriction zone comprising a second determined nucleotide sequence and a second blunt-ended cleavage site at a second determined distance of said second nucleotide sequence,

- a determined sticky-ended restriction site outside of both first and second tele-acting restriction zones

wherein

- said first and second nucleotide sequences are positionned with respect to each other such as to provide for said first and second cleavage sites to be distant of a selected number of 1 or more nucleotides from each other, and

- said sticky-ended restriction site lying outwards of both said first and second restriction zones.

In the following, the expression "tele-acting enzyme" is used to designate restriction enzymes which cleave a DNA fragment located itself at a determined distance from a specific nucleotide sequence or site recognized by the said teleacting enzyme. Thus such "teleacting" enzyme permits a DNA fragment not to be cleaved at the level of the site specifically recognized, rather at a fixed distance downstream (or upstream) of said site. In other words the "restriction zone" referred to hereabove comprises the sequence specifically recognized by the tele-acting enzyme and the cleavage site itself.

The distance between first and second cleavage sites depends on the tele-acting enzyme chosen. By using preferred enzymes disclosed hereafter, first and second cleavage sites will be distant from each other of one, or two nucleotides.

But it should be noted that these particular enzymes are given hereafter by way of example, and that

5

other "tele-acting" enzymes can be selected, which aim at providing first and second cleavage sites of said tele-acting enzymes to be distant of another number of nucleotides.

Examples of teleacting enzymes are XmnI and of MnlI.

In preferred vectors disclosed hereafter, the abovesaid "first teleacting enzyme" consists of XmnI which exerts its action on a "first teleacting restriction zone" having the following nucleotidic sequence :

```
                  *
    ---G-A-A-N-N-N-T-T-C---
    ---C-T-T-N-N-N-A-A-G---
                  *
```

wherein (1) C, G, A, T are the usual abbreviations of cytosine, guanosine, adenine and thymine, and N is any nucleotide, (2) the "first determined sequence" is the one described above with the appropriate spacing and, (3) the cleavage under the action of the teleacting enzyme occurs between the second and third N downstream of GAA in order to provide said first "blunt-ended cleavage site" (at the level of the "*"sign). Thus, this first "split palindrome or tele-acting" enzyme exerts its action by splitting its recognition sequence with arbitrary base pairs as described above.

In an analogous manner a preferred "second tele-acting enzyme" consists of MnlI which exerts its action on a "second tele-acting restriction zone" having the following nucleotidic sequence :

```
                      +
    ---C-C-T-C-N-N-N-N-N-N-N-N---
    ---G-G-A-G-N-N-N-N-N-N-N-N---
                      +
```

wherein (1) C, G, A, T are the usual abbreviations of cytosine, guanosine, adenine and thymine, and N is any

6

nucleotide, (2) the "second determined sequence" is CCTC
GGAG
and, (3) the cleavage under the action of the teleacting enzyme occurs between the seventh and eighth N downstream of CCTC, in order to provide said second "blunt-ended cleavage site" (at the level of the "+" sign).

Thus preferred vectors of the invention comprise the following region :

```
                         +     *
----C-C-T-C-N-N-N-N-G-A-A-N-N-N-N-T-T-C-----
----G-G-A-G-N-N-N-N-C-T-T-N-N-N-N-A-A-G-----
                         +     *

    -------          -----          -----
    MnlI             XmnI---------XmnI
```

For the easiness of language, the expression "blunt-ended cleavage site" is used to designate a cleavage site which when cleaved by the relevant restriction enzyme, results in the production of blunt-ended DNA fragments.

Thus the sites marked by the "*" and "+" signs in the above examplified DNA-region define the first and second "blunt-ended cleavage sites" associated with XmnI and MnlI respectively. In addition it will be appreciated that in the examplified region the abovesaid "first" and "second" blunt-ended cleavage sites lie two nucleotides apart. Reference will be made later to the "DNA element" consisting of the 1, or more nucleotides which lie between said first and second blunt-ended cleavage sites.

In an analogous way, the expression "sticky-ended cleavage site" designates a site which, when cleaved by the appropriate enzyme, results in DNA having sticky ends.

7

Again by way of example the following sequence
-G-G-T-N-A-C-C-
-C-C-A-N-T-G-G-
is a "sticky-ended cleavage site" specifically recognized by the BstEII restriction enzyme.

Vectors (phages or plasmids) according to the invention are useful, in combination with a DNA sequence, hereafter called module, for the production of polynucleotides, by stepwise additions of single base pairs or small fragments to one another.

The method of the invention, using said vectors and module, is based on an insertion - cloningdeletion strategy resulting in a stepwise elongation of the module with successive nucleotides in the order called for by the final sequences desired in the polynucleotides sought.

Thus the invention aims also at providing a method for producing at one blunt end of a module, an oligonucleotide or polynucleotide of any desired nucleotide structure by a "non-chemical route". The oligonucleotide formed may then be separated from the module (provided that a suitable restriction site for a specific restriction enzyme or teleacting enzyme has been formed at the fixation site of the oligonucleotide to the module) or not. The first alternative (i.e. separation of the oligonucleotide from the module) is of particular interest, for instance when it is purported to obtain an oligonucleotide consisting of a linker containing a specified restriction site sought in the laboratory. The second alternative is applicable in any instance when the module consists itself of a DNA fragment (or part thereof) which must be elongated with an oligonucleotide including a specified restriction site, for instance for subsequent recombination in vitro with another DNA comprising a same specified restriction site.

8

The module which can be used in combination with the above vectors consists of any nucleotide sequence devoid of the second tele-acting restriction zone corresponding to that of the vectors and possessing at one of its extremities the same sticky-ended restriction site as that of the vectors, and a blunt end at its opposite extremity.

The process of the invention for elongating a module by a selected number of 1 or more determined nucleotides - or of a DNA fragment or sequence as defined hereabove whose first nucleotides correspond to said selected number of one or more nucleotides - at one of its extremities comprises, starting from said module with a blunt end at one extremity and with a sticky end at its opposite extremity,

- cleaving the appropriate vector (that vector which comprises said selected number of determined nucleotides between its above-defined first and second cleavage sites) in said first tele-acting restriction zone with the corresponding first teleacting enzyme and in the said determined sticky-ended restriction site with the corresponding restriction enzyme (thereafter called "third restriction enzyme") thereby obtaining a first vector fragment comprising a blunt end and another second vector fragment comprising a sticky end,

- ligating said module with said vector fragments to provide a modified vector having said module inserted between the abovesaid first and second vector ends,

- cleaving said modified vector in said second teleacting restriction zone with the corresponding teleacting enzyme and again with the restriction enzyme in the sticky-ended restriction site which had been reformed in the modified vector, and

- recovering the module of elongated the

9

selected number of 1 or more nucleotides supplied by the modified vector.

The successive results obtained at the end of each cycle of the sequence of operations defined hereabove can be summed up as follows.

The cleavages achieved in the starting vector with the first tele-acting enzyme and the third restriction enzyme, followed by the above mentioned ligation of the module allow for the substitution of the module for a fragment initially located in the starting vector between the first blunt-ended cleavage site associated with the first teleacting enzyme and the third restriction site associated with the third restriction enzyme.

The cleavages achieved in the modified vector obtained at the end of the preceding sequence of operations with the second teleacting enzyme and again the third restriction enzyme enable the excision and recovery of the module from the modified vector. Yet the module is itself now modified in that it further includes at its blunt extremity an elongation by the selected number of nucleotides, i.e. the "DNA element" initially present in the starting vector.

It will be understood by the man skilled in the art that this result is achieved in asmuch as the first, second and third cleavage sites of the starting vector are located in the appropriate order. Particularly the reversal of the order of the first and second cleavage positions with respect to the third one may to the contrary result in the shortening (rather than elongation) of the module by one or more nucleotides.

It will be apparent to the man skilled in the art that the module so elongated forms a new module which can again be elongated at one of its extremities by any DNA element comprising 1 or more nucleotides. In other words the new module constitutes the starting module for another elongation cycle, using the same procedure with another vector as defined herein and so forth.

The repetition of this process results in a stepwise elongation of the starting module, said elongation always occurring at the same extremity of the starting module.

However it will be appreciated that the new module will again be elongated by a DNA element identical to the preceding one if the same starting vectors were used. But a distinct DNA element or "building block" can be used for the further elongation of the module, with another vector similar to the preceeding one, yet comprising a "DNA-element" or building block comprising the selected number of nucleotides between its first and second cleavage sites, which are sought to be added to the modified module.

Therefore the invention also relates to the sets or "kits" of vectors comprising all possible "building blocks" which are required to enable the user to synthesize any oligo- or poly- nucleotide having any desired nucleotidic sequence, subject to carrying out the appropriate series of synthesis cycles.

Obviously a set of four vectors in which the DNA elements or "building blocks" located between the "first" and "second" blunt-ended positions consist of the four distinct single pairs of nucleotides, will be suitable for the one-by-one stepwise synthesis of any polynucleotide.

A more speedy sequence of operations can be achieved with a set of vectors including "building blocks" corresponding to all possible dinucleotide base pairs. But this more speedy sequence then involves the availability of 16 vectors, in order to encompass all possibilities that may be required.

11

Obviously an even more speedy production of polynucleotides could be achieved with vectors including "building blocks" with three base pairs each. the feasible length of these building blocks depends on the first enzyme used. But then 64 vectors would be required for encompassing all possibilities, a number which may render corresponding kits particularly cumbersome.

Thus a practical compromise lies in sets of vectors comprising, respectively :

- 16 vectors with "dinucleotide building blocks" and

- 4 vectors with "mononucleotide building blocks".

The invention thus concerns "kits" of vectors, including particularly the above sets of vectors and, particularly, if the synthesis of separate oligonucleotides is sought, at least one module.

Preferred kits further comprise also part or all of the following items :

- the required "first" and "second" teleacting enzymes and "third" restriction enzyme,

- the reagents required for preparing the media enabling the necessary in vitro recombinations and, optionally,

- hosts, particularly bacteria, for instance in lyophilized or other suitable form capable of providing for the replication of the vectors of the invention,

- culture media or nutrients, for said competent hosts.

Irrespective of whether the synthesized oligonucleotide is to be separated from the module or not, it should be noted that the module should carry, at one extremity, a blunt end, and, at the opposite extremity, a nucleotide sequence containing the same sticky-ended

12

restriction site as that of the vectors, thus a restriction site recognized by the above-mentioned third restriction enzyme. Such module (whether elongated or not) will then always be inserted into the vectors of the invention in the proper orientation, said insertion then resulting in the complete reconstruction of said third restriction site.

Advantageous modules are those which also contain a sequence recognized by a "blunt cleaving" enzyme (distinct of those associated with the vectors), said sequence being then positionned such as to provide the cleavage of the synthesized oligonucleotide or polynucleotide at the level of the blunt end of the module, before elongation thereof.

For the easiness of language, the expression "blunt-cleaving" enzyme is used to designate a restriction enzyme which cleaves a DNA fragment and results in the production of blunt-ended DNA fragments.

As concerns the vectors it must be understood that they are not subject to any particular condition, except that of being capable to carry a region having the different sites which have been contemplated herein and of being free of other sites which could seriously interfere with their use in the synthesis method in accordance with the invention. Obviously they should comprise the suitable replicons for authorizing the cloning of these vectors in the selected hosts, particularly E. coli.

Further it will become obvious to the man skilled in the art that the invention brings up a new technical principle for the direct synthesis of double-stranded nucleic acid sequences, accordingly that the disclosure of the examples which follows should not be considered in a limitative manner, particularly as concerns the specific sequences and sites designated herein. It goes without saying that the man skilled in the
art is capable of devising other vectors (plasmids, phages or cosmids) with other appropriate sites, capable of
carrying out similar functions.

Additional advantages of the invention will
appear in the following disclosure of one preferred embodiment, given by way of example in the light of the
drawings in which :

Figure 1 represents the plasmid pGV403, given
by way of example, which is used for the construction of
the vectors of the invention. In particular, the positions and numbers of different restriction sites are indicated.

Figure 2 represents the plasmid pGV532, given
by way of example, used in the construction of the vectors of the invention. Restriction sites are indicated
as above.

Figure 3 represents plasmid pGV451 derived
from pGV403 and pGV532, which bears the chloramphenicol
acetyltransferase gene downstream of the beta-lactamase
promoter of plasmid pBR322.

Numbers and positions of the different restriction sites are indicated.

Figure 4 represents plasmid pGV454 derived
from pGV451, after insertion therein of a XhoI linker
containing two tele-acting restriction zones.

Figure 5 represents plasmid pSYN00 derived
from pGV454 obtained in figure 4 and which contains the
two tele-acting restriction zones and the sticky-ended
restriction site.

Figure 6 shows the number and location of the
different restriction sites present in the plasmid
pSYN00 of figure 5.

0245130

14

In the above figures, restriction sites are indicated as follows : starting from the periphery to the center of the figure, each one of the six circles represents one of the following restriction sites : XmnI, MnlI, BstEII, EcoRI, HindIII and TTM111I respectively.

In each circle corresponding to one of the above said restriction sites, the location of each restriction site is indicated by a line which cuts the circle, and the number of above said restriction sites present in the plasmid corresponds to the number of lines cutting the circles corresponding to said restriction sites.

Embodiments disclosed hereafter illustrate the experiments carried out by the Applicant to achieve a stepwise addition of determined dinucleotides at one extremity of a module. It also relates to the construction of the sixteen vectors according to the invention, necessary for said stepwise dinucleotide-additions.

Four enzymes are used in the synthesis process described herebelow :

They represent one way to develop the synthesis system. Several other potential combinations are feasible by suitable experimental programming and construction of corresponding vectors.

The enzymes used in this prototype systems are T4 DNA-ligase and three restriction enzymes : XmnI, MnlI and BstEII. XmnI represents the first tele-acting enzyme and is used for insertion of a module into the vector (i.e. the cloning enzyme) ; MnlI represents the second tele-acting enzyme and is used for the cleavage of the module at the elongating side (i.e. the excision enzyme) ; BstEII represents the sticky-ended restriction enzyme and is used for excision at the opposite side of the module, as well as for cloning : this results in an

0245130

15

oriented ligation ('forced cloning') of the module into the vectors.

BstEII can readily be replaced by other enzymes, depending on the vector system. It can also be dispensed with if only a very limited number of synthesis steps is envisaged ; in that case selections by direct DNA sequencing is possible after each cycle.

As concerning more particularly the BstEII restriction enzyme, reference can be made to the German Patent Application n°3.314.994.1. filed on April 4, 1983, which is incorporated herein by reference.

Reference can also be made to the scientific literature, particularly to the following article : VOLCKAERT et al, Gene.Anal.Techn. ; 1 : 52-59, 1984, which is also incorporated herein by reference. This latter article relates to a rapid chemical DNA sequencing system in which the BstEII enzyme is used.

Principle of synthesis :

A set of 16 vectors has been constructed, each of them containing "DNA elements" or "building blocks" corresponding to the 16 possible combinations of dinucleotides respectively. These vectors contain the following sequence :

```
                          *    +
-------C-C-T-C-N-N-N-N-G-A-A-N-N-N-N-T-T-C---------
-------G-G-A-G-N-N-N-N-C-T-T-N-N-N-N-A-A-G---------
                          *    +

    -------           -----           -----
    MnlI              XmnI---------XmnI
```

The "-N-N-" dinucleotide between the "*" and "+" signs corresponds to the 16 dinucleotide pairs distinct from one another.

In addition, 4 vectors comprising the 4 possible single base-pair building blocks between the "*" and "+" signs were prepared. They are characterized by a

16

region :

```
                                            *  +
-------C-C-T-C-N-N-N-G-A-A-N-N-N-N-T-T-C-------
-------G-G-A-G-N-N-N-C-T-T-N-N-N-N-A-A-G-------
                                            *  +
```

The construction of preferred sets of vectors will be described later.

There follows beforehand the disclosure of a synthesis cycle using a determined module and one of the above said vectors.

The module is thereafter diagrammatized by :

```
            pN------G
               N------C-C-A-G-T-Gp          .
```

in which p is phosphate.

Vectors contained a third restriction site, namely a BstEII restriction site, whereby the following order of restriction sites were present in the vectors :

```
        ---MnlI---XmnI---BstEII---
```

corresponding to the following nucleotide sequence :

```
--C-C-T-C-N-N-N-N-G-A-A-N-N-N-N-T-T-C----G-G-T-C-A-C--
--G-G-A-G-N-N-N-N-C-T-T-N-N-N-N-A-A-G----C-C-A-G-T-G--
```

This third BstEII enzyme recognizes and cleaves the nucleotide sequence as follows :

```
            *
        -G-G-T-N-A-C-C-
        -C-C-A-N-T-G-G-
                    *
```

The cleavage site is indicated by the sign "*".

The first step of the synthesis cycle using a determined module and the above-said vectors was :

1) Cleavage of the vector with the XmnI enzyme The following fragments were formed :

```
--C-C-T-C-N-N-N-N-G-A-A-N-N pN-N-T-T-C----G-G-T-C-A-C---
--G-G-A-G-N-N-N-N-C-T-T-N-Np N-N-A-A-G----C-C-A-G-T-G---
```

in which Np represents the 5' end, and a terminal N the 3' end (p being phosphate).

This first step was completed by another cleavage of the vectors with the BstEII enzyme.

This step in the synthesis cycle is performed such that the module be ligated in the correct orientation, i.e. that the same end of the module is always at the same (here the left) side of the vector. This may be achieved in a variety of manners, for example by having a transcriptional promoter in the module and a promoterless selectable gene in the vector or vice-versa. The cleaving enzyme BstEII provides the selective orientation to be achieved.

Thus, after double digestion of the vector at its unique XmnI and BstEII restriction sites, the ends of the cleaved vector fragments were as follows :

```
----C-C-T-C-N-N-N-N-G-A-A-N-N        pG-T-C-A-C-C----
----G-G-A-G-N-N-N-N-C-T-T-N-Np               G---
```

The second step of the synthesis cycle consisted in the :

2) Insertion of the restriction fragment module with T4 DNA-ligase into the modified vector :

After *in vitro* recombination the following recombinant was recovered :

```
--C-C-T-C-N-N-N-N-G-A-A-N-N-N------G-G-T-C-A-C-C---
--G-G-A-G-N-N-N-N-C-T-T-N-N-N------C-C-A-G-T-G-G---
```

in which the fragment corresponding to the module is indicated.

3) Excision of the elongated module from the vector.

The recombinant was first cleaved by the MnlI enzyme to provide fragments having the following ends respectively :

```
---C-C-T-C-N-N-N-N-G-A-A       N-N-N------G-G-T-C-A-C-C---
---G-G-A-G-N-N-N-N-C-T-T       N-N-N------C-C-A-G-T-G-G---
```

18

Finally, the excision of the module was completed by the cleavage with BstEII.

Thus, a modified vector was finally obtained which was elongated on its left side and exhibited the following formula (as compared with the starting module) :

pN-N-N------G

N-N-N------C-C-A-G-T-Gp

Be it recalled that it is essential that the module does not contain a MnlI recognition sequence.

Any fragment bearing a blunt end at one side and a BstEII protruding end at the other side fulfils the necessary criteria to act as a module.

In the above example, the BstEII restriction site was located on the right side of the two tele-acting restriction zones, whereby the elongation of the module took place at the left extremity thereof.

However, it should be noted that said elongation could also have been carried out at the right extremity of said module, the BstEII restriction site been located into the vector on the left side of the two tele-acting restriction zones, as follows :

---BstEII-----XmnI----MnlI----

This second case is envisaged in the following construction of preferred sets of vectors. Thus having obtained a first elongation of the module, an additioned one can then further be obtained upon using the elongated module obtained as a new module in a new synthesis cycle. It must be understood that the vector used in the new synthesis cycle is then selected according to the building block sought to be added in the further elongated module.

The synthesis cycles are then further repeated, each time with the appropriate vectors (containing "building blocks" successively formed either of the mononucleotide - or dinucleotide - base-pairs), selected in accordance with the final sequence sought in the synthesized final oligonucleotide or polynucleotide.

CONSTRUCTION OF A SET OF VECTORS,(pSYNOO) AUTHORIZING A STEPWISE DINUCLEOTIDE ADDITION.

Several series of vectors can be elaborated, the construction of the 4 and 16 vector sets is discussed here, and given by way of example. It goes without saying that the man skilled in the art is capable of devising other plasmids with appropriate sites, capable of carrying similar functions. The first step was the construction of plasmid pGV451 which is a rapid chemical sequencing plasmid deriving from pGV403 and pGV532.

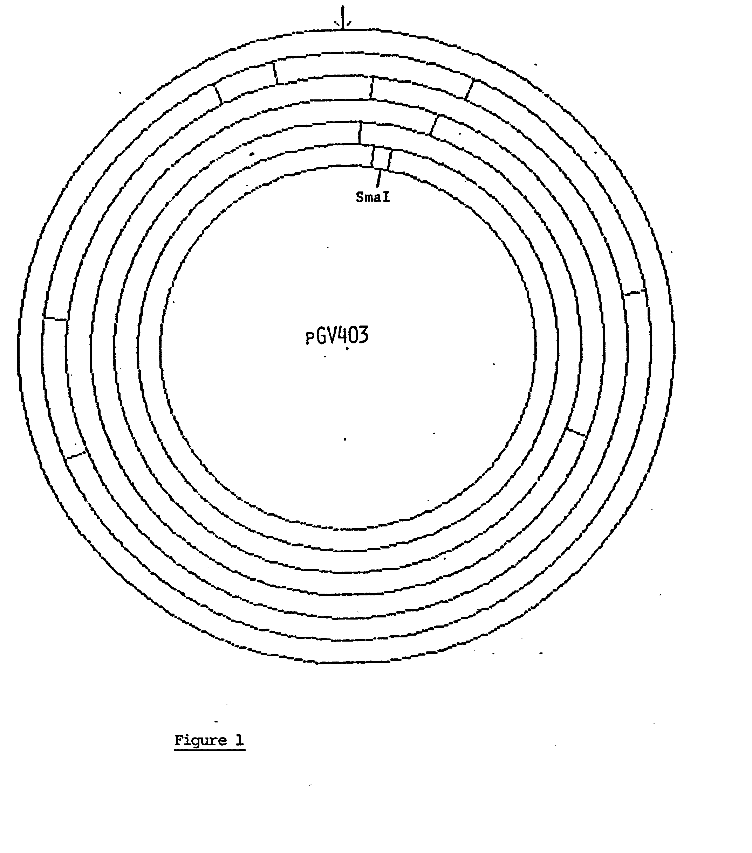

Plasmid pGVO43, as shown in figure 1, comprises no XmnI site, 6 MnlI sites, 1 BstEII site, 2 EcoRI sites, 1 HindIII site and 2 TTH111I sites.

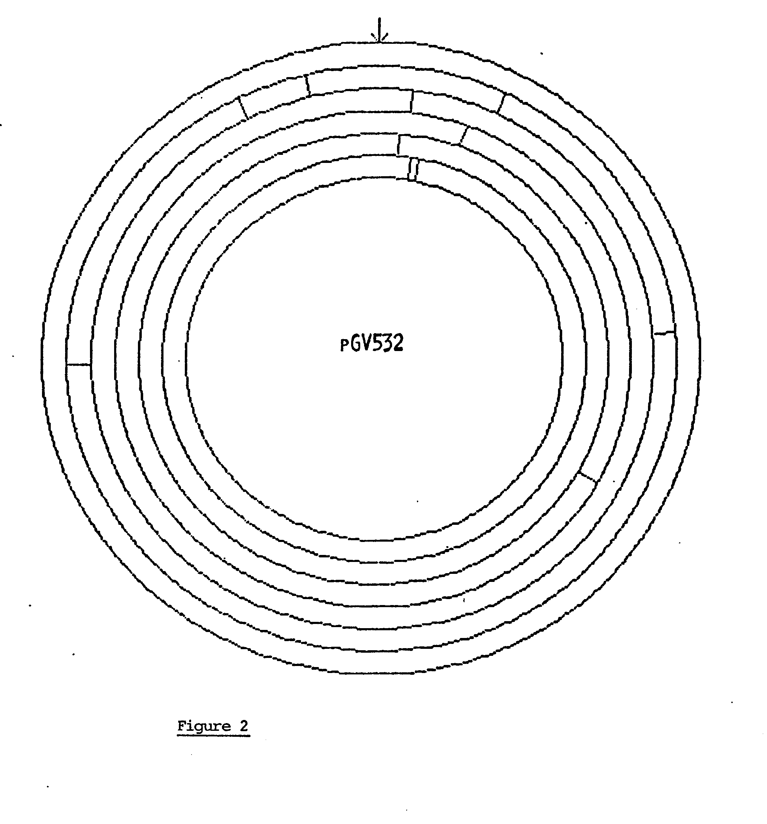

Plasmid p6V532, as shown in figure 2, comprises no XmnI sites, 5 MnlI sites, 1 BstEII site, 2 EcoRI sites, 1 HindIII site and 2 TTH111I sites.

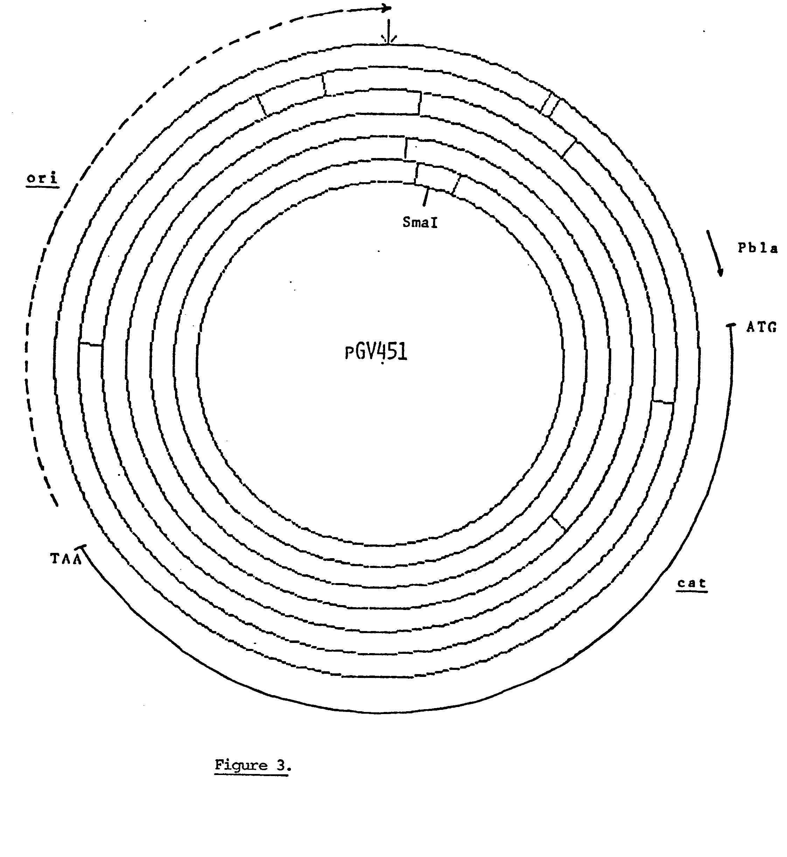

Figure 3 shows that plasmid pGV451 bears the chloramphenicol acetyltransferase gene (cat) devoid of its promoter downstream of the beta-lactamase promoter (Pbla) of pBR322 (Bolivar et al, Gene. Vol.2, p.95, 1977). Hence, it confers resistance to chloramphenicol. In addition, it contains a rapid sequencing configuration as mentioned above.

Plasmid pGV451 comprises 2 XmnI sites, 5 MnlI sites, 1 BstEII site, 1 EcoRI, 1 HindIII site and 2 TTH111I sites.

Ori corresponds to the promoterless region (Col E) essential for replication.

A new derivative was then made by cloning of a commercial XhoI linker (Biolabs, Beverly, USA) into the

20

SmaI restriction site of pGV451.

This new plasmid was named pGV454, and comprises 2 XmnI sites, 7 Mnl I sites, 1 BstEII site, 1 EcoRI site, 1 HindIII site and 2 TTHM111I sites.

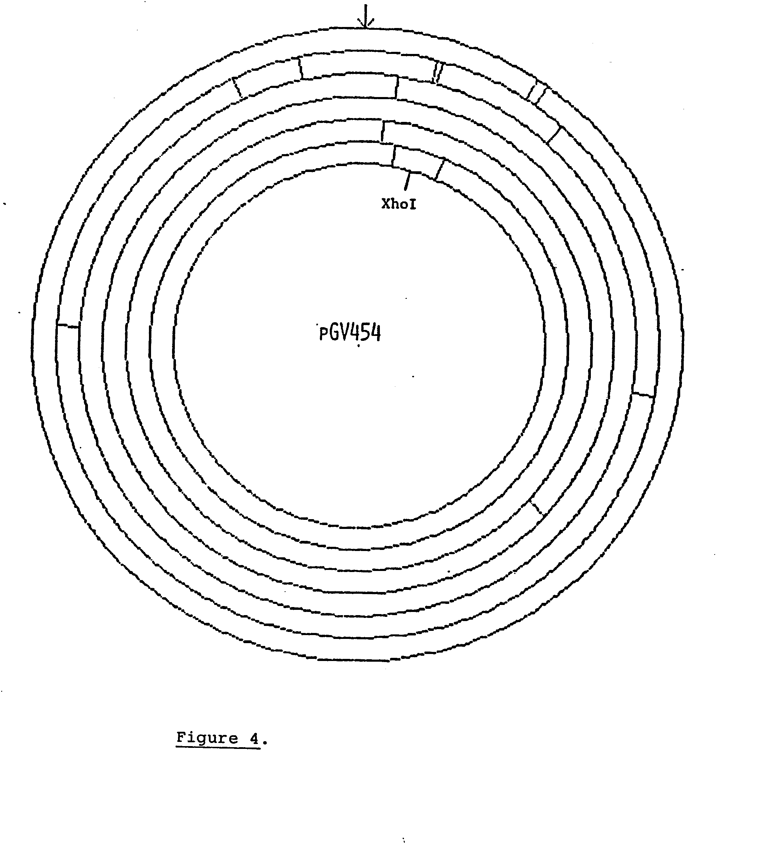

pGV454 is diagrammatized in figure 4. The XhoI linker is 8 nucleotides long and comprises the following sequence :

C-C-T-C-G-A-G-G-

It is formed by two MnlI sites :

C-C-T-C

and

G-A-G-G,

this latter sequence corresponding to the complementary strand of C-C-T-C.

Subsequently, two sets of oligonucleotides were chemically synthesized :

G-A-A-T-T-N-N-T-T-C-G-G

and

C-C-G-A-A-N-N-A-A-T-T-C

in which "N" represents any nucleotide.

Thus, a mixture of sixteen double-stranded linker fragments was formed by mixing both series :

G-A-A-T-T-N-N-T-T-C-G-G

C-T-T-A-A-N-N-A-A-G-C-C

in which the dinucleotide -N-N- was different in each linker fragment.

These fragments were inserted into the filled-in XhoI restriction site of pGV454 and resulted in the following set of sixteen vectors which comprise the following sequence (only one orientation is shown here) :

G-G-T-G-A-C-C-C-C-T-C-G-A-G-A-A-T-T-N-N-T-T-C-G-G-T-C-G-A-G-G-

C-C-A-C-T-G-G-G-G-A-G-C-T-C-T-T-A-A-N-N-A-A-G-C-C-A-G-C-T-C-C-

BstEII       MnlI        XmnI      ·     MnlI

21

These sixteen plasmids differ from each other only by a dinucleotide, located between the XmnI and MnlI sites, as shown hereafter.

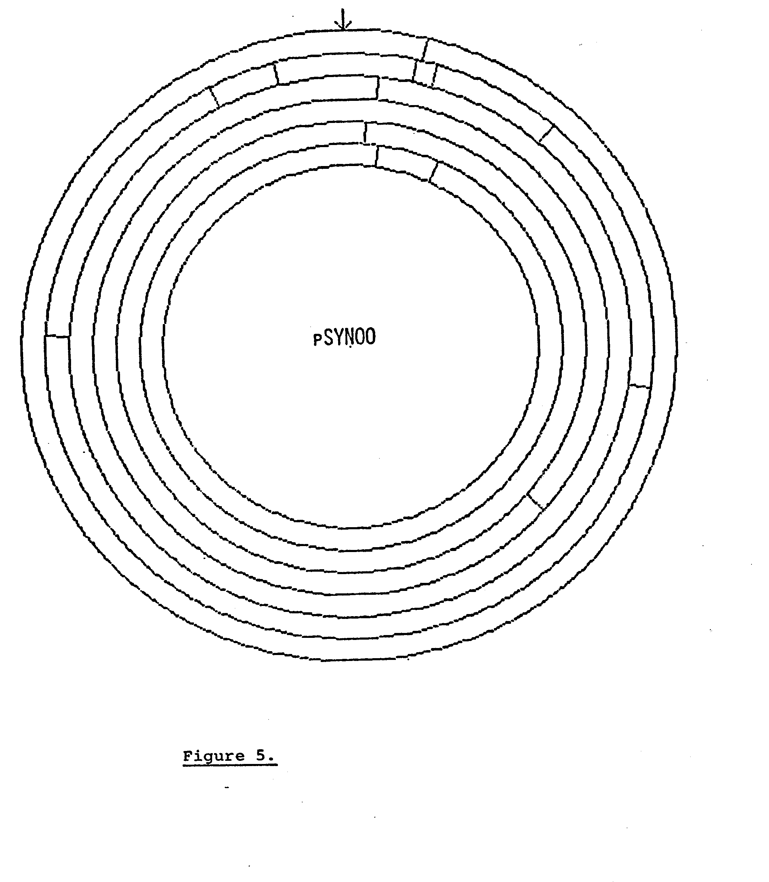

These plasmids, which can be generally designated by plasmid pSYNOO, in which the -N-N- dinucleotide corresponds to any of the sixteen dinucleotides shown hereafter, comprise 1 XmnI site, 7 MnlI sites, 1 BstEII site, 1 EcoRI site, 1 HindIII site and 2 TTH111I sites, as shown in figure 5.

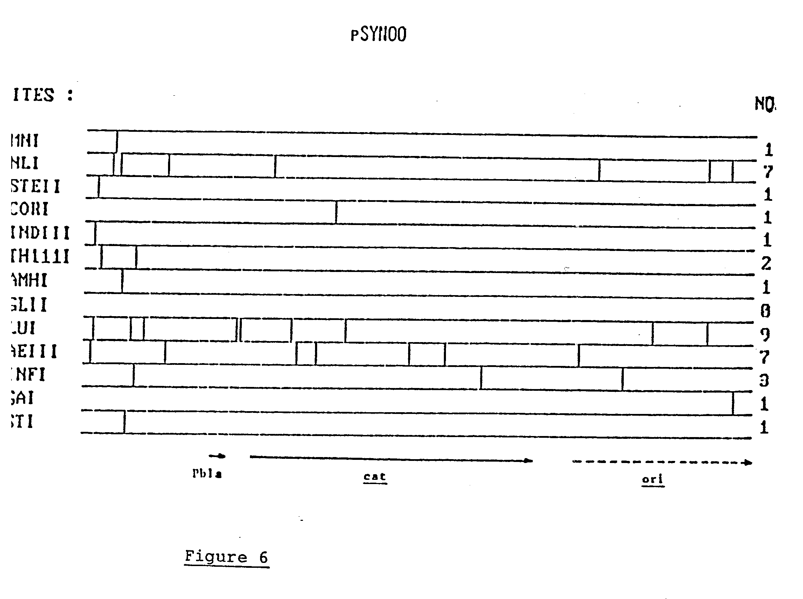

A restriction map of pSYNOO is also represented in figure 6, in which Pbla, cat, and ori have the same significations as already indicated in figure 3.

Another XmnI restriction site obtained from pGV454 (which is also a BglII site), was removed by cleavage with the BglII enzyme, filling-in of the sticky ends and self-ligation.

The vectors were named as follows (upper strand is only shown) :

Only the dinucleotide -N-N- differing in each vector is represented thereafter.

```
----A-A----------------------------------- :pSYN17
----A-G----------------------------------- :pSYN18
----A-C----------------------------------- :pSYN19
----A-T----------------------------------- :pSYN20
----G-A----------------------------------- :pSYN21
----G-G----------------------------------- :pSYN22
----G-C----------------------------------- :pSYN23
----G-T----------------------------------- :pSYN24
----C-A----------------------------------- :pSYN25
----C-G----------------------------------- :pSYN26
----C-C----------------------------------- :pSYN27
----C-T----------------------------------- :pSYN28
----T-A----------------------------------- :pSYN29
----T-G----------------------------------- :pSYN30
----T-C----------------------------------- :pSYN31
----T-T----------------------------------- :pSYN32
```

In addition, four plasmids were constructed which allow a single-base-pair addition per synthesis cycle. They originate from incomplete filling-in of the XhoI restriction site during the cloning step of the linkers into plasmid pGV454.

They were named :

pSYN37 : addition of the single base pair : A

pSYN38 : addition of the single base pair : G

pSYN39 : addition of the single base pair : C

pSYN40 : addition of the single base pair : T

This original sixteen vectors series has been extended to a second series containing selection marker between the BstEII and XmnI restriction sites.

This selection marker is the strS gene which confers streptomycin sensitivity to streptomycin resistant E. coli cells carrying the vector.

23

Thus, we select against remnants of uncleaved or partially cleaved vector during the synthesis procedures to avoid background colonies of vector.

strS gene is obtainable from the commercially available plasmid pNO1523 (Pharmacia-PL-Biochemicals).

The strS gene was cloned into the EcoRI site of pSYN25, whereafter it was transferred into the above described plasmids pSYN17 to pSYN40, as a BstEII-XmnI fragment.

This leads to the series pSYN117 to pSYN140, which has the same synthesis configuration of the corresponding pSYN17 to pSYN40 vectors.

EXAMPLE OF A DETERMINED DNA FRAGMENT SYNTHESIS.

For a better understanding, only upper strand is shown thereafter, but it should be noted that in reality, a double-stranded DNA fragment was synthesized.

The fragment to be synthesized was as follows:

G-G-G-A-A-T-C-A-A-C-C-G-T-C-G-G

The BstEII-SmaI fragment of plasmid pGV403, which contains the following sequence :

G-T-G-A-C-C-C-T-G-A-C-T-A-A-G-T-C-G-A-G-C-C-C

was used as the module in this synthesis cycle.

Step 1 :

Plasmid pSYN122 was cleaved with the enzymes BstEII and XmnI. The module was then substituted for the BstEII-XmnI fragment of pSYN122.

After cleavage of pSYN122 with the enzymes BstEII and MnlI, and excision of the relevant fragment, the module was elongated at its right extremity with the dinucleotide G-G as follows :

G-T-G-A-C-C-C-T-G-A-C-T-A-A-G-T-C-G-A-G-C-C-C-G-G

In the following, this elongated module will be represented by :

X-X-X-G-G

24

Step 2 :

Plasmid pSYN121 was cleaved with the enzymes BstEII and XmnI. The module obtained in step 1 was then substituted for the BstEII-MnlI fragment of pSYN121.

After cleavage of pSYN121 with the enzymes BstEII and MnlI, and excision of the relevant fragment, the nucleotide sequence of the module was elongated with the dinucleotide G-A as follows :

X-X-X-G-G-G-A

Step 3 :

Plasmid pSYN120 was cleaved with the enzymes BstEII and XmnI. The module obtained in step 2 was then substituted for the BstEII-XmnI fragment of pSYN120.

After cleavage of pSYN120 with the enzymes BstEII and MnlI, and excision of the relevant fragment, the module was elongated with the dinucleotide A-T as follows :

X-X-X-G-G-G-A-A-T

Step 4 :

Plasmid pSYN125 was cleaved with the enzymes BstEII and XmnI. the module obtained in step 3 was then substituted for the BstEII-XmnI fragment of pSYN125.

after cleavage of psyn125 with the enzymes BstEII and MnlI, and excision of the relevant fragment, the module was elongated with the dinucleotide C-A as follows :

X-X-X-G-G-G-A-A-T-C-A

Step 5 :

Plasmid pSYN119 was cleaved by the enzymes BstEII and XmnI. The module obtained in step 4 was then substituted for the BstEII-XmnI fragment of pSYN119.

After cleavage of pSYN119 with the enzymes BstEII and MnlI, and excision of the relevant fragment, the module was elongated with the dinucleotide A-C as follows:

25

X-X-X-G-G-G-A-A-T-C-A-A-C

Step 6 :

Plasmid pSYN126 was cleaved with the enzymes BstEII and XmnI. The module obtained in step 5 was then substituted for the BstEII-XmnI fragment of pSYN126.

After cleavage of pSYN126 with the enzymes BstEII and MnlI, and excision of the relevant fragment, the module was elongated with the dinucleotide C-G as follows :

X-X-X-G-G-G-A-A-T-C-A-A-C-C-G

Step 7 :

Plasmid pSYN131 was cleaved with the enzymes BstEII and XmnI. The module obtained in step 6 was then substituted for the BstEII-XmnI fragment of pSYN131.

After cleavage of pSYN131 with the enzymes BstEII and MnlI, and excision of the relevant fragment, the module was elongated with the dinucleotide T-C as follows:

X-X-X-G-G-G-A-A-T-C-A-A-C-C-G-T-C

Step 8 :

Plasmid pSYN122 was cleaved with the enzymes BstEII and XmnI. The module obtained in step 7 was then substituted for the BstEII-XmnI fragment of pSYN122.

After cleavage of pSYN122 with the enzymes BstEII and MnlI, and excision of the relevant fragment, the module was elongated with the dinucleotide G-G as follows:

X-X-X-G-G-G-A-A-T-C-A-A-C-C-G-T-C-G-G

The synthesized fragment was then excised from the final construction by combined cleavage with SmaI and MnlI.

Another use of the invention is of particular significance, that is for the synthesis at will of oligonucleotides or polynucleotides coding for selected peptides or polypeptides. This may be achieved

26

particularly when carrying out the above-disclosed process upon a set of 20 vectors of the type defined hereabove, wherein said 20 vectors include between their respective first cleavage sites and second cleavage sites three-nucleotide-fragments encoding the 21 natural aminoacids, respectively in combination with a suitable module.

It should be understood that in order to provide for the elongation of the module, the first cleavage site under the control of the first determined nucleotide sequence of the first tele-acting restriction zone should be located downstream of the second cleavage site with respect to the second determined sequence of the tele-acting restriction zone. Particularly in the examples described hereabove under the subtitle "Principle of synthesis" (in which the first cleavage site and second cleavage site were distant by 2 nucleotides from each other) it will be appreciated that the first cleavage site (controlled by the XmnI site) was located 2 nucleotides downstream of the second cleavage site (controlled by the MnlI site) with respect to the second determined sequence; i.e. C-C-T-C (or G-G-A-G).

It is also to be noted that in that example, the first determined nucleotide sequence of the first tele-acting zone, i.e. G-A-A (or C-T-T) was separated through 4 nucleotides from the second determined nucleotide sequence of the second tele-acting zone, i.e. from C-C-T-C (or G-G-A-G)/

If by contruction G-A-A is brought in continguous contact with C-C-T-C, it will be appreciated that the resulting vector used in combination with the same module and by carrying out the same steps as those which have been disclosed above, will result in the final shortening of the module by two pairs of nucleotides, whatever the two nucleotides between said first cleavage

27

site and said second site are. If by construction of the vector one leaves a single nucleotide between the G-A-A and C-C-T-C site, the use of such vector under the conditions discussed hereabove will result in the shortening of the module by one nucleotide only.

The invention thus also provides for a process which permits the controlled removal of successive "determined numbers n" of nucleotides at one of the extremities of any DNA used as a module. This method can be of particular significance, for instance in assays performed on DNA sequences consisting of open reading frames encoding a protein, in order to study and determine the sequence encoding epitopes responsible of the immunogenic properties of said protein, such assays then comprising cloning successively shortened fragments (particularly by one or several codons) in a microorganism under conditions permitting the expression of said fragments, such as by detecting the expression products by antibodies against that protein or to the contrary, by testing the immunogenic properties of the shortened peptide obtained in the suitable in vivo assay systems.

0245130

28

## CLAIMS

1. A vector applicable to an enzymatical synthesis of a determined nucleotide fragment, which comprises :

- a first tele-acting restriction zone comprising a first nucleotide sequence and a first blunt-ended cleavage site at a first determined distance of said first nucleotide sequence,

- a second tele-acting restriction zone comprising a second nucleotide sequence and a second blunt-ended cleavage site at a second determined distance of said second nucleotide sequence,

- a determined sticky-ended restriction site, wherein

- said first and second nucleotide sequences are positionned with respect to each other such that said first and second cleavage sites are distant of a selected number n of nucleotides from each other, wherein n is an integer which is not zero, and

- said sticky-ended restriction site lies outwards of both said first and second restriction zones.

2. Vector according to claim 1, wherein the distance n between said first and second cleavage sites is 1, 2 or 3 nucleotide-long.

3. Vector according to claim 1 or 2, wherein said first tele-acting restriction zone is a XmnI restriction zone which comprises the following nucleotidic sequence :

       -G-A-A-N-N-N-N-T-T-C-
       -C-T-T-N-N-N-N-A-A-G-

in which N represents any one of the four bases G,A,T,C.

4. Vector according to any one of claims 1 to 3, wherein said second tele-acting restriction zone is a MnlI restriction zone which comprises the following nucleotidic sequence :

29

-C-C-T-C-N-N-N-N-N-N-N-

-G-G-A-G-N-N-N-N-N-N-N-

in which N represents any one of the four bases G, A, T, C.

5. Vectors according to any one of claims 1 to 4, wherein said sticky-ended restriction site is a BstEII restriction site which comprises the following nucleotidic sequence :

-G-G-T-N-A-C-C

-C-C-A-N-T-G-G

in which N represents any one of the four bases G, A, T, C.

6. A process for making the enzymatical synthesis of a sought nucleotide fragment which, upon using appropriate vectors according to any one of claims 1 to 5 and wherein all of said vectors include a common determined sticky-ended restriction site and a common ————————————— second tele-acting zone , which process comprises cycles of operations, and wherein said starting module does not contain the second tele-acting zone of said vectors, each of said cycles comprising :

- starting from a module provided with a blunt end at one extremity and with a sticky end at its opposite extremity, which sticky end is complementary to a sticky end included in said common determined sticky-ended restriction site of said vectors,

- cleaving an appropriate vector in its first tele-acting restriction zone with the corresponding first tele-acting enzyme and in its determined sticky-ended restriction site with the corresponding restriction enzyme and excising the vector fragment located between said first tele-acting restriction zone and said sticky-ended restriction site, said vector being chosen such as to contain between said first and second cleavage sites of said first and second tele-acting restriction zones the n nucleotides which constitute the first n nucleotides of the sought nucleotide fragment, whereby the cleaved vector thus obtained provides vector-fragments which can be ligated to the opposite extremities of said module,

- ligating said starting module with said vector-fragments and recovering said vector modified by the insertion of said module therein, which modified vector then contains a common sticky-ended restriction site reformed therein,

- cleaving said modified vector in said second teleacting restriction zone with the corresponding second tele-acting enzyme and again with the restriction enzyme cleaving said common restriction site, said cleavage allowing for said vector and an elongated module comprising the starting module with an elongation of n nucleotides, to be separated from each other,

- recovering the elongated module comprising an elongation of n nucleotides of the determined nucleotide sequence sought at its blunt end,

and

- repeating said cycle of operations on said elongated module which has then become a new starting module, if the determined nucleotide sequence sought is longer than said elongation, with another appropriate vector, said vector being chosen such that the n nucleotides located between the first and second cleavage sites of said first and second tele-acting restriction zones correspond to the next n nucleotides of the sought nucleotide sequence which come immediately after the n nucleotides already added to said starting module during the first cycle, said second cycle of operations allowing for said elongated starting module to be elongated further at the same extremity with said n next other nucleotides,

31

- repeating, whenever necessary, said cycles of operations on the further elongated module until the starting module has been elongated by the full determined nucleotide sequence sought.

7. The process of claim 6 wherein the terminal portion of the module which is to be elongated by said determined nucleotide sequence sought consisted of a determined additional restriction site cleavable by the corresponding blunt-cleaving restriction enzyme at the extremity of said terminal portion to provide a blunt extremity, which process further comprises cleaving the final module with said additional blunt-cleaving restriction enzyme and recovering said determined nucleotide sequence sought.

8. Process according to claim 6 or 7 wherein the distance n between said first and second cleavage sites is 1, 2 or 3 nucleotide-long.

9. Process according to any one of claims 6 to 7, wherein said first tele-acting restriction zone is a XmnI restriction zone which comprises the following nucleotidic sequence :

   -G-A-A-N-N-N-N-T-T-C-

   -C-T-T-N-N-N-N-A-A-G-

in which N represents any one of the four bases G,A,T,C.

10. Process according to any one of claims 6 to 9, wherein said second tele-acting restriction zone is a MnlI restriction zone which comprises the following nucleotidic sequence :

   -C-C-T-C-N-N-N-N-N-N-N-

   -G-G-A-G-N-N-N-N-N-N-N-

in which N represents any one of the four bases G, A, T, C.

11. Process according to any one of claims 6 to 10, wherein said sticky-ended restriction site is a BstEII restriction site which comprises the following

32

nucleotidic sequence :

        -G-G-T-N-A-C-C

        -C-C-A-N-T-G-G

in which N represents any one of the four bases G, A, T, C.

12. A process for making the enzymatical synthesis of a sought nucleotide fragment which , upon using appropriate vectors according to any one of claims 1 to 5 and wherein all of said vectors include a common determined BstEII restriction site and a common MnlI restriction zone, wherein process comprises cycles of operations, and which said starting module does not contain the MnlI restriction zone of said vectors, each of said cycles comprising :

    - starting from a module provided with a blunt end at one extremity and with a sticky end at its opposite extremity, which sticky end is complementary to a sticky end included in said common determined BstEII restriction site of said vectors,

    - cleaving an appropriate vector in its first XmnI restriction zone with the corresponding first XmnI enzyme and in its determined BstEII restriction site with the corresponding BstEII restriction enzyme and excising the vector-fragment located between said XmnI restriction zone and BstEII restriction site, said vector being chosen such as to contain between said first and second cleavage sites of said XmnII and MnlI restriction zones the n nucleotides which constitute the first n nucleotides of the sought nucleotide fragment, whereby the cleaved vector thus obtained provides vector-fragments which can be ligated to the opposite extremities of said module,

    - ligating said starting module with said vector fragments and recovering said vector modified by the insertion of said module therein, which modified vector then contains a common BstEII restriction site

33

reformed therein

- cleaving said modified vector in said second MnlI restriction zone with the corresponding second MnlI restriction enzyme and again with the BstEII restriction enzyme cleaving said common BstEII restriction site, said cleavage allowing for said vector and an elongated module comprising the starting module with an elongation of n nucleotides, to be separated from each other,

- recovering the elongated module comprising an elongation of n nucleotides of the sought nucleotide fragment at its blunt end,

and

- repeating said cycles of operations on said elongated module which has then become a new starting module, if the determined nucleotide sequence sought is longer than said elongation, with another appropriate vector, said vector being chosen such that the n nucleotides located between the first and second cleavage sites of said XmnI and MnlI restriction zones correspond to the next n nucleotides of the sought nucleotide fragment which come immediately after the n nucleotides already added to said starting module during the first cycle, said second cycle of operations allowing said elongated starting module to be elongated further at the same extremity with said n next other nucleotides,

- repeating, whenever necessary, said cycles of operations on the further elongated module until the starting module has been elongated by the full determined nucleotide sequence sought.

13. The process of claim 12 wherein the terminal portion of the module which is to be elongated by said determined nucleotide sequence sought consisted of a SmaI restriction site cleavable by the corresponding SmaI restriction enzyme at the extremity of said terminal portion to provide a blunt extremity, which

0245130

34

process further comprises, cleaving the final module with said SmaI enzyme and recovering said determined nucleotide sequence sought.

14. A set comprising four vectors, according to any one of claims 1 to 5, wherein said two cleavage sites are separated from each other by one mononucleotide building block, said mononucleotide building block being different from one vector to all others.

15. A set comprising sixteen vectors according to any one of claims 1 to 5, wherein said two cleavage sites are separated from each other by one dinucleotide building block, said dinucleotide building block being different from one vector to all others.

16. Kit applicable to an enzymatical synthesis of a determined nucleotide sequence which comprises :

- a set comprising four vectors according to claim 14,

- a set comprising sixteen vectors according to claim 15,

- first and second tele-acting enzymes according to claim 3 or 4 respectively, and a third restriction enzyme corresponding to a sticky-ended restriction site according to claim 5,

- a module provided with a blunt end at one extremity and with a sticky end at its opposite extremity, which sticky end is complementary to the determined sticky-ended restriction site contained in said vectors and which module does not contain the second tele-acting zone of said vectors.

Sma I

pGV403

Figure 1

PGV532

Figure 2

Figure 3.

XhoI

pGV454

Figure 4.

0245130

PSYN00

Figure 5.

6/6

pSYN00

Figure 6

0245130

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 068 701 (CANADIAN PATENTS AND DEVELOPMENT LTD) * Page 6; page 15, last paragraph; page 16 * | 1-3 | C 12 N 15/00 C 12 P 19/34 |
| | --- | | |
| A | GENE ANALYSIS TECHNIQUES, vol. 1, January 1984, pages 52-59, Elsevier Science Publishing Co. Inc., New York, US; G. VOLCKAERT et al.: "A novel type of cloning vectors for ultrarapid chemical degradation sequencing of DNA" * Figure 2 * | 1,5 | |

TECHNICAL FIELDS SEARCHED (Int. Cl.4)

C 12 N
C 12 P

The present search report has been drawn up for all claims

| Place of search | Date of completion of the search | Examiner |
|---|---|---|
| THE HAGUE | 06-07-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82